# EUROPEAN PATENT APPLICATION

(11) **EP 2 336 770 A1**
(43) Date of publication of application: **22.06.2011**
(21) Application number: 10180591.9
(22) Date of filing: 10.06.2005
(51) Int. Cl.: G01N 33/50, G01N 33/48, G06F 19/00

(54) **Methods for identifying conditions affecting a cell state**

(30) Priority: 12.08.2004 US 600964 P
(62) Divisional of application: 05760335.9
(71) Applicant: Transform Pharmaceuticals, Inc., Lexington, MA 02421 (US)
(72) Inventor: Levinson, Douglas, Sherborn, MA 01770, MA (US); Kitsos, Christine, Chelmsford, MA 01824 (US); Melnikova, Irena, Jefferson, MA 01522 (US); McNulty, Christopher, Arlington, MA 02474 (US)
(74) Representative: Hallybone, Huw George

(57) **Abstract**

The present invention is directed to methods of identifying agents which affect cell state. The instant invention provides rapid and efficient methods for identifying agents which affect cell state. Methods are directed toward the screening of complex combinations of agents for their ability to affect cell state. In one embodiment, cells are incubated under suitable conditions and subjected to different agents. After an appropriate amount of time, the cells are assayed to determine what, if ant characteristics they possess. Cell characteristics can be organised in a manner such that different and novel cell states can be identified.

## Description

### CROSS-REFERENCE TO RELATED APPLICATIONS

The present application claims the benefit of priority of U.S. Provisional Application Serial No. 60/600,964, filed August 12, 2004, the contents of which are incorporated herein by reference in their entirety.

### FIELD OF THE INVENTION

This invention generally relates to methods of identifying one or more agents. In particular, this invention pertains to methods of identifying conditions that promote, permit, inhibit or maintain certain cell states.

### BACKGROUND OF THE INVENTION

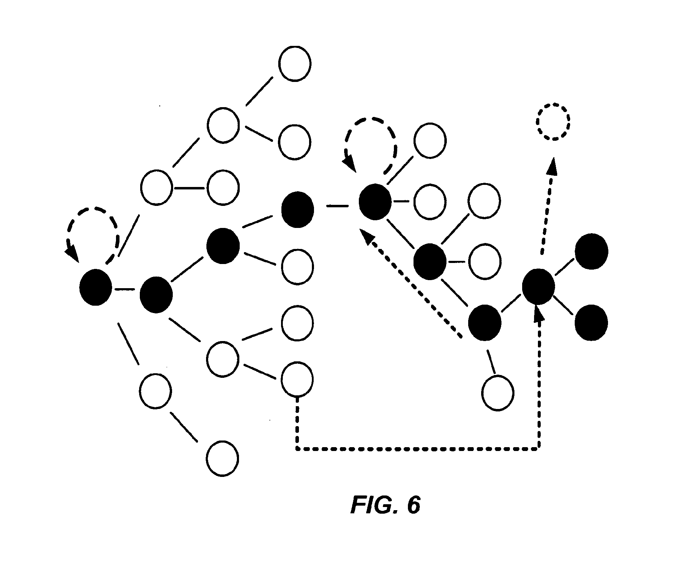

Differentiated cells begin their life as pluripotent cells, typically referred to as stem cells. Pluripotent stem cells are cells that have not yet been assigned a particular phenotype. The term "pluripotent" refers to this ability, *i.e*., the ability of a stem cell to differentiate into any number of mature cells. For example, the stem cells of the bone marrow can form red blood cells or white blood cells depending upon the chemical milieu these cells are exposed to. Particular factors or sets of factors act upon the stem cell directing it toward a certain phenotype. Depending upon which factors or set of factors act upon a particular stem cell, the stem cell will differentiate into a mature committed cell, such as a T lymphocyte. Often it is not just one factor that determines the fate of a stem cell. Typically, it is a combination of factors that promote a particular path of differentiation. Figure 6 illustrates a typical differentiation process from a stem cell. Thus, the cells can have multiple different pathways. The arrows indicate potential places for cells to differentiate, dedifferentiate, transdifferentiate, or regenerate.

Certain populations of cells often play a critical role in, for example, in fighting disease. For example, white blood cells as a whole are employed by the body to fight bacterial, viral, and parasitic invasions. Within the white blood cell grouping there are specific cells that have particular functions. For example, the specific immunity system is composed of lymphocytes. Lymphocytes can be either T lymphocytes or B lymphocytes. The former can be subdivided again into, for example, cytotoxic and helper T cells. The latter B lymphocytes can further differentiate into memory cells or plasma cells (cells that elaborate soluble proteins called antibodies). All of these cells have specific phenotypes associated with them; they may also share some characteristics as well. Some of the phenotypic characteristics are particular antigens elaborated on the surface of a differentiated cell. For example, T-helper cells elaborate an antigen called CD4, while cytotoxic T cells elaborate an antigen CD8. These antigens allow for a method of broadly identifying a cell population. Even though some cell types can be identified by one marker, a population of cells expressing one marker may not all function identically. Thus, not all CD8 cytotoxic T cells will respond to antigen equally. A population of CD8 cytotoxic T cells will consist of cells in varying cell states even though all the cells may express CD8. Thus, all the cells of a population may be positive for CD8, but they may vary in expression of multiple other markers or the secretion of factors. It would be desirable to identify the different cell states of CD8 expressing cells to determine what factors produce this cell state and how this cell state is different from other cell states. Likewise, it is desirable to characterize cell state for many other cell types.

The cells of the immune system, antigen specific or not, go through cell differentiation. Cellular factors influence the specific path of differentiation. Some of the factors associated with particular cellular differentiation have already been elucidated. In a therapeutic setting, it is desirable to have the ability to isolate stem cells and direct them toward a certain end, for example, the production of T-helper cells. Although single factors influencing differentiation or small combinations of factors influencing differentiation have been identified, complex combination of factors or conditions which affect cell differentiation and cell state have not been characterized. For example, it may be known that interleukin 6 causes differentiation effects on CD8 cytotoxic T cells, but it is not known what mix of factors create CD8 cytotoxic T cells which have the highest response to antigen.

For some clinical conditions, taking an undifferentiated cell towards a differentiated cell may not be desirable. It may also desirable to de-differentiate a cell, e.g. go from a differentiated cell to an undifferentiated cell. Few single cellular factors involved in this process are known. Cellular de-differentiation is likely to involve a complex mix of cellular factors and not one or two single factors can be easily identified. In other clinical conditions, it may be desirable to cause transdifferentiation, e.g., go from one type of differentiated cell to another type of differentiated cell. This process can change one mature cell into another mature cell that has a different phenotype. Similarly, cellular transdifferentiation is likely to involve a complex mix of cellular factors and not one or two single factors which can be easily identified.

Traditional exploration into elucidating the various cellular factors responsible for cellular differentiation often is tedious and labor intensive. In addition, traditional high throughput screening for factor activity involves testing single factors. Testing multiple factors can be very difficult for a system which not only test cell differentiation, but also tests for cell state. For example, if you test 5 different factors at three different concentrations combined in ternary under two different temporal dimensions, you would have 7,110 possible experiments if you looked at one output. The number of experiments would increase to 28,440 if you were looking at four outputs. Testing a complex combination of factors and looking for multiple cell states in specific types of cells cannot be accomplished with traditional pharmaceutical high throughput systems. Current high throughput systems are typically limited in either input variety or output variety. Thus, current systems do not adequately address the large experimental space relating to cell state. A new type of system is needed which can analyze complex input and output data and group the data into a readable format.

Each of the techniques mentioned above must be coupled with a form of data analysis and handling techniques to enable data collection and processing of hundreds or thousands of samples. These and other difficulties are overcome by the methods disclosed herein.

### SUMMARY OF THE INVENTION

The present invention is directed to methods for identifying agents which affect cell state. In particular, the instant invention provides rapid and efficient methods for identifying agents which affect cell state.

In one embodiment, methods are directed toward the screening of complex combinations of agents for their ability to affect cell state. In this embodiment, cells are incubated under suitable conditions and subjected to different agents. After an appropriate amount of time, the cells are assayed to determine what, if any, characteristics they possess. Cell characteristics can be organized in a manner such that different and novel cell states can be identified.

In one embodiment, methods for identifying a cell state comprise providing an array of receptacles each containing cells to be investigated, subjecting said cells in different receptacles to different agents, waiting a pre-determined period of time before analyzing said cells, analyzing said cells for expression of markers, creating a spectra representing marker expression of a cell population, and grouping said cell populations with similar spectra. In one aspect, the grouping is represented in a plot or graph. In another aspect, the cell state occurs in the presence of at least three different treatment conditions, five different treatment conditions, eight different treatment conditions, or ten different treatment conditions. In another aspect, the treatment conditions vary by the agent or agents added to the receptacles. In a further aspect, the cell state occurs in the presence of at least three different treatment conditions. In some aspects, the grouping occurs with the aid of a computer or the grouping occurs by calculating the Euclidean distance between the spectra of different treatment conditions and ordering the measures of distance of all spectra in a Tartan plot based on similarity. In another embodiment, said method further comprises the steps of plotting cells with similar expression markers in profile comprising a first axis representing at least two markers, and a second axis representing the number of positive cells expressing said markers.

In one embodiment, the agents vary in one or more variables selected from the following: a first agent variable relating to the number of agents added to said receptacle; a second agent variable relating to the timing of the addition of the agent into a receptacle; a third agent variable relating to the amount or concentration of agent added to a receptacle; a fourth variable relating to the identity or type of agent added into a receptacle; and a fifth variable relating to the period of time an agent is present in a receptacle.

In another embodiment, a method of identifying a cell state comprises providing an array of receptacles each containing cells to be investigated, subjecting said cells in different receptacles to different agents, waiting a pre-determined period of time before analyzing said cells, analyzing said cells for expression of markers, creating a spectrum representing marker expression of a cell population; and grouping said cell populations with similar spectra wherein said cell states are defined by at least 25 different treatment conditions which have similar profiles. In one aspect, the cell states are identified by at least 50 or 100 different treatment conditions which have similar profiles. In a further embodiment, the similar profiles are determined by calculating the Euclidean distance between the spectra of different treatment conditions; and ordering the measures of distance of all spectra in a Tartan plot based on similarity.

In one embodiment, a method of identifying a cell state comprises the steps of providing a cell population, introducing a set of agents to the cell population, detecting a set of markers, and creating a profile. In one aspect, the cell population can be a heterogeneous or homogeneous cell population. In another aspect, the cell population can be animal cells or plant cells. In another aspect, the cell population is derived from cells selected from the group consisting of epithelial cells, endothelial cells, stem cells, mesenchymal cells, fibroblasts, neuronal cells, hematopoietic cells, and progenitor cells. In another aspect, the cell populations are essentially in the same cell cycle.

In one embodiment, the set of agents comprises at least 2, at least 3, at least 5, at least 10, at least 15, between 2 and 20, between 4 and 10, between 2 and 8, or between 5 and 12 agents. In one aspect, the agents are introduced at one or more concentrations. In another aspect, the agents are soluble factors; insoluble factors; cell matrix components; proteins; peptides; carbohydrates; small molecules; inorganic molecules; organic molecules, conditioned media, cell extracts, tissue extracts, pH modifiers, gasses, osmotic pressure modifiers; ionic strength modifiers; viruses; DNA; RNA; gene fragments; temperature modulators; mechanical stress modulators; or pressure modulators.

In one embodiment, the marker is expression of a certain molecule; secretion of a certain agent; a specific phenotype; loss of a specific molecule; a change in membrane permeability; a change in electrical potential; cell death; cell migration; cell differentiation; gene expression changes; changes in protein levels; phosphorylation; methylation; or acetylation. In one aspect, the profile comprises at least two markers, at least three markers, at least five markers, at least 10 markers, at least 15 markers, between 2 and 20 markers, between 4 and 10 markers or between 3 and 8 markers. In another aspect, the detection marker is an antibody, receptor; ligand, antisense molecule, small molecule, and reporter construct.

In one embodiment, a method of creating a profile comprises the steps of choosing a set of markers, detecting said markers on a population of cells, and creating a graphical representation of the percent of cells expressing a particular marker. In another embodiment, a method of inducing a specific cell state comprises the steps of identifying a desired cell population, creating a specific profile for said desired cell population; creating cell population induced with a set of agents, identifying a profile for said cell population induced with said set of agents, and comparing said specific profile for said desired cell population to said profile for said cell population induced with said set of agents. In a further embodiment, a method of identifying conditions which induce a specific cell state comprises the steps of identifying a desired cell population, creating a specific profile with a specific set of markers for said desired cell population, incubating a cell population with a set of agents, identifying a profile with the same set of markers for said cell population induced with said set of agents, and comparing said specific profile for said desired cell population to said profile for said cell population induced with said set of agents. In an additional embodiment, a profile comprises an axis representing at least two markers, and an axis representing percent positive cells responding to said markers. In one aspect, the percent positive cells are calculated by averaging the percent of cell expressing a particular marker from at least two test populations of cells.

### BRIEF DESCRIPTION OF THE DRAWINGS

Figure 1 illustrates (a) a typical histogram and (b) a profile formed from the histograms;

Figure 2 are three examples of profiles (a, b, & c);

Figure 3 is a matrix generated from multiple profiles;

Figure 4 is a view of the matrix data showing groups of similarities;

Figure 5 is a diagram of an exemplary process of this invention;

Figure 6 is a diagram of a typical stem cell differentiation pathway;

Figure 7 is a graphical representation of HL-60 marker expression;

Figure 8 is graphical representation of HL-60 cells selected for myeloid marker expression;

Figure 9 is a profile comparing factor dominance;

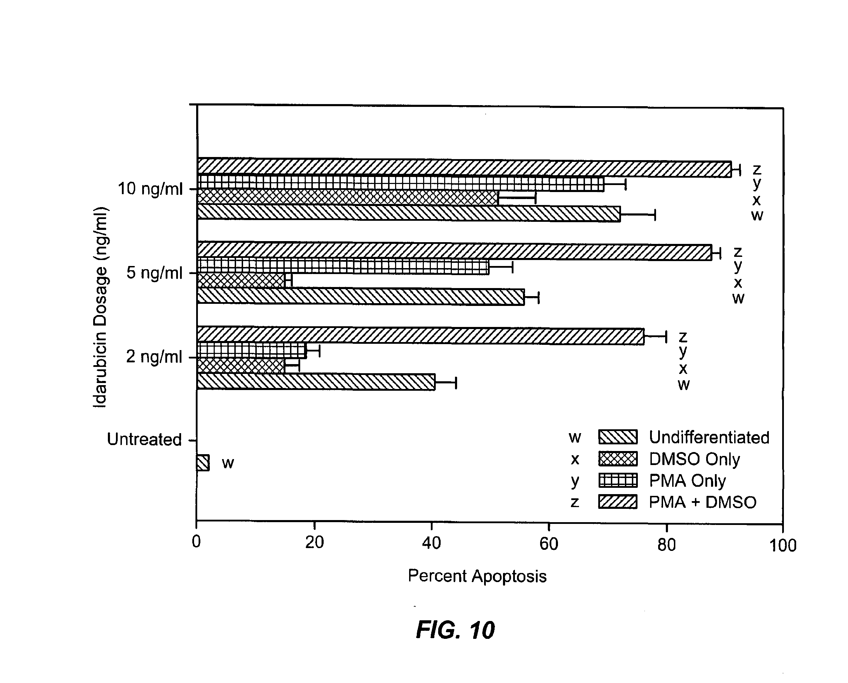

Figure 10 is a chart comparing HL-60 cell rate apoptosis after treatment with idarubicin and additional agents; and

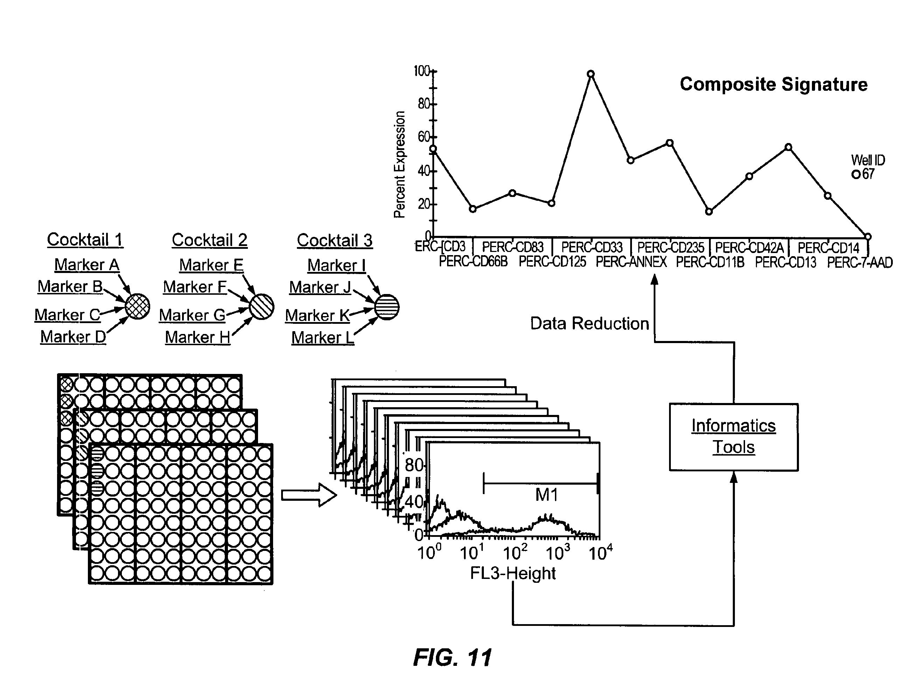

Figure 11 is a representation of the type of data used in making a profile.

### DETAILED DESCRIPTION OF THE INVENTION

The present invention is directed to methods for identifying agents which affect cell state. In particular, the instant invention provides rapid and efficient methods for screening complex conditions which promote, permit, inhibit or maintain a certain cell state. Additionally, the invention provides for methods that are used to screen conditions that modulate certain biological responses, such as cell death, cell differentiation, cell proliferation, receptor expression, gene expression, cell responsiveness to stimuli, and alike.

For purposes of this invention, "agent" includes, but is not limited to, soluble factors, insoluble factors, cell matrix components, proteins, peptides, carbohydrates, small molecules, inorganic molecules, organic molecules, conditioned media, cell extracts, tissue extracts, pH modifiers, gasses, osmotic pressure modifiers, ionic strength modifiers, viruses, DNA, RNA, gene fragments, temperature modulators, mechanical stress modulators and pressure modulators.

For purposes of this invention, "complex conditions" is defined to be conditions under which at least one cell type is subjected to at least two different agents.

For the purposes of this invention, "marker" is defined to be a type of identification for a cell state. A marker can be expression of a certain molecule, secretion of a certain agent, a specific phenotype, loss of a specific molecule, a change in membrane permeability, a change in electrical potential, cell death, cell migration, cell differentiation, gene expression changes, changes in protein levels, phosphorylation, methylation, acetylation, or any other characteristic which can be used to differentiate between different cell states.

For purposes of this invention, "cell state" means a condition of a cell where the cell expresses specific factors, responds in a particular way to agents, has a specific metabolic profile, has a specific gene or protein expression profile, or has a specific morphology.

Embodiments of this invention can: 1) quantify the effects of combinatorial treatment using multiple observable variables and link these to experimental design information; b) observe system-wide patterns of response for different markers, and be able to explore patterns of interest; c) detect nonlinear, non-obvious effects between differentiation factors in a combinatorial space; d) identify novel formulations that control differentiation; and e) detect unexpected cell states.

The present invention employs the use of cells. These cells can be obtained from fresh tissue, they can be from an immortalized cell line and alike. The cell system can comprise a homogenous cell population. Alternatively, the cell system can comprise a heterogenous population of cells. Cells can be of the same origin and state of differentiation. Alternatively, the cells can be of the same origin yet differ in their state of differentiation. In another aspect, the cells can be of different origin. For example, stem cells can be derived from an embryo or from bone marrow. The cells can be homogeneous with respect to their phenotype or they can be heterogenous with respect to their phenotype as evidenced by elaborating different cell surface antigens. The cells used can be in different mitotic phases or can be synchronized. In one aspect, the cells have a short cycle.

The cell sample employed can be plant or animal cells or a combination thereof. In one aspect, a host cell line comprises the following characteristics: they have a short cycle (*i.e*., around 20-36 hour doubling time), amenable to high throughput procedures without undue loss of membrane integrity or viability, susceptible to standard techniques designed to introduce various agents, including proteins, peptides, nucleic acids, carbohydrates and a combination thereof. An elaboration of these characteristics can be found in WO 03/008648. In one aspect, the cells used posses a deficiency, genetic or otherwise, indicative of a diseased state.

Typical cells which can be used in this invention are available from the American Tissue Culture Company. Any of the cells available from the American Tissue Culture Company could be used in this invention. For example, a typical cell line that can be employed is the HL60 promyelocytic leukemia cell (ATCC# CCL-240). Other cells appropriate for this invention include, but are not limited to, Hs 855.T, HS-5, 2E8, HCN-2, FCH, J26, HF 345.We, 293, WI-38, CTLL, and TM3. The breadth of this invention is not limited by the type of cells used. For example, cells can be derived from epithelial cells, endothelial cells, stem cells, mesenchymal cells, fibroblasts, neuronal cells, hematopoietic cells, embryos, and others.

In one example, the assay is a non-destructive assay (e.g., a cell-based assay in which a measurement of the effect of an agent can be obtained without harming the cells). Such an assay allows multiple combinations per well. For example, agent A is added at increasing concentrations to a well and a marker measurement is taken after each addition of agent. When a desired concentration of agent A is reached (determined based on a desired assay response or on known properties - such as toxicity, solubility and alike, of the factor), agent B is added in increasing concentrations, with a marker measurement taken after each addition. This process can be iterated many times in a single well (or using multiple wells), allowing hundreds, thousands, or even millions of assays to be performed in a single plate.

Comparison assays can be used to identify possible biological effects of complex conditions. For example, combinations of agents that are screened for their ability to elicit a particular biological response, such as expression of a particular marker, can be simultaneously screened. Obviously, those combinations that produce the desired effect are preferred, while those combinations that do not produce the desired effect are less preferred. It is important to note, however, that the less preferred combinations may generate the desired effect at a different concentration. Combinations of agents may produce synergistic effects or produce effects which are different than the agents could produce individually.

In one aspect of the present invention, a cell extract can be used as the experimental system. The cell extract can contain all of a cell's components but lack an integral cell membrane and/or wall. Therefore, the cell's contents can be exposed to whatever experimental conditions placed in contact with the cellular contents. The experimental system can comprise only certain components of a cell. Preparation of these various experimental systems are well known to those skilled in the art.

Cell culturing techniques for transformed and non-transformed cells are well known in the art. The cells can be cultured and stored until required for use. The media used for culturing can be specifically designed, or alternatively, commercial sources of media can be purchased.

The platform used in the present invention comprises one or more receptacles that can receive cells and culture media. For example, a 96 well plate is a platform that can be used in the instant invention. Other multi-well platforms are also within the scope of this invention. Analogous structures can also be used, for example 1.5 mL tubes can be used. Any receptacle suitable for holding and sustaining cells is within the scope of this invention. One preferable characteristic of the containment vehicle is that it allows for analysis, be it spectrophotometric analysis or any other well known analytical method. However, this is not a critical limitation as the solution contained within a given platform can be transferred to a suitable platform amenable to further analysis. In one aspect, the platform is amenable to the addition of a protective covering, thus protecting against the entry of contaminants.

Complex conditions can be examined to determine what affect, if any, they have on an intact cell. The experimental conditions can vary based on their agents and/or the concentration of the agents. One aspect of the invention is directed to variations based on differences in agent composition. For example, condition 1 can comprise, retinoic acid, interleukin 6 and interleukin 11, while condition 2 comprises dimethylsulfoxide, growth hormone and nerve growth factor. Agents can include other classes of molecules as well. Another aspect of this invention includes varying the concentration of one or more agents. For example, three different concentrations (high, medium, and low) can be used to study a range of agent concentration effects. Thus, in one embodiment, the concentration of agent is tested in a high, medium and low concentration of agent. One skilled in the art could identify the specific high, medium, and low concentrations for each specific agent.

The differences between various conditions can also be differences in temporal and spatial dimensions. Differences in temporal administration of agents can affect cell state. For example, administration of IL-12 to T cells causes the upregulation of the CD28 immune regulator receptor. Thus, adding B7.1 (the ligand for CD28) after upregulation of CD28 could have a different effect than adding B7.1 at the same time as the IL-12 or before the expression of the CD28. Thus, variation in temporal administration of agents can identify different cell states and identify the agents which promote, permit, inhibit or maintain those cell states. In one aspect of this invention, the order of agent administration is varied. Agents can be added minutes apart, hours apart, or even days apart.

Variations in spatial administration of the agents can also affect cell state. For example, activating antibody bound to the surface of a bead or a plate is known to activate cells differently than freely soluble antibody. Thus, the spatial manner of presentation can affect the cell state. Examples of special differences are where agents can be freely soluble, can be attached to a plate, can be attached to another rigid surface, can be agglomerized, can be put into small spaces, can be put into large spaces, or can be layered.

A cocktail is a composition comprising one or more detection molecules that is specific for a particular predetermined marker or set of markers. Cocktails can contain 1, more than 1, more than 2, more than 3, more than 5, more than 10, more than 15, or more than 20, different detection markers. Cocktails can differ in their constituents. For example, cocktail 1 contains antibodies A, B & C, while cocktail 2 contains antibodies D, E & F. Each of these antibodies is specific and directed toward a particular marker. Cocktails that detect receptors can be composed of antibodies, ligands, or other molecules which can bind to the desired receptor. In other embodiments, a cocktail may comprise a marker gene such as green fluorescent protein. Cocktails that detect DNA or RNA could consist of nucleic acid binding molecules. Thus, the components of a cocktail can vary depending upon the specific marker which is being explored.

Multiple arrays of experimental conditions can be realized by exposing cells to multiple agents or multiple combinations of agents (including environmental changes such as pH, ionic strength, *etc*. ). Each of the individual conditions and cocktails can be added to individual units (*e.g*., wells) within the platform. For example, assume that a 96-well plate is the platform being utilized. Also, assume that there are three different agents (A, B, & C) to be examined. Additionally, assume that a cocktail comprises antibodies X, Y & Z and will be used to detect the presence (or absence) of certain markers attendant to the cell population examined. Assuming the experiment is performed in triplicate, plate 1 will comprise, for example, cells in three different wells, the same is true for plate 2 and plate 3. To plate 1 is added condition A, to plate 2 is added B, and to plate 3 is added C. To plates 1,2 & 3 is added the cocktail comprising antibodies X, Y & Z. The cells are incubated for a sufficient period of under suitable conditions and then subjected to analysis. Histograms can then be produced from conducting analysis of the defining markers. Once the histograms are produced, the information contained therein can be transformed into various other forms of data representation, such as a graph. Agents A, B, and C can also be tested in various combinations.

The agents used in an experiment may or may not have known biological function/activity. Some agents will function similarly such that synergistic relationships can be found. Other agents will have different functions. In one aspect, the same agent can be tested with multiple different concentrations or ratios of agent.

Additionally, other non-chemical, factors can be screened in combination with a specific set of agent. Non-chemical factors can include, but not limited to, light (visible and outside the visible range, *e.g*., infrared and ultraviolet light), ionizing radiation such as X-rays and gamma-rays, hyperbaric pressure, increased or decreased temperature or pH, gaseous substances such as oxygen, nitrogen, carbon dioxide, and alike, and acoustic vibrations of any frequency.

The assay itself can be based on an individual cellular component, such as the presence or absence of an antigen, alternatively, it can be based upon a biological response, such as a change in second messenger, or electrical activity. Any biological assay that is useful for assay of individual or combinatorial agents is readily adapted to the present invention. Assay measurements can include, for example, transport of a compound across the cell membrane, electrical potential, action potential generation, cell proliferation, cell death, cell specification, cell differentiation, cell migration, gene expression or protein levels (measured, e.g., by detecting mRNA, protein, or a reporter gene), enzymatic activity, phosphorylation, methylation, acetylation, translocation of a protein to the cell nucleus (or other changes in protein locus, such as translocation of a protein from the cytosol to the cell surface), ability to resist a pathogenic challenge, ability to respond to an agent, and ability to produce an immune response.

The method of detection can vary. Any detection system which can detect the applicable markers can be used. Detection markers function to detect a specific marker. Detection markers include, but are not limited to, antibodies, receptors, ligands, antisense molecules, small molecules, and reporter constructs (such as green fluorescence protein). The detection marker used will vary depending upon the biological assay being conducted and the marker which is desired.

For example, if the assay is directed toward the differentiation of a cell from a stem cell into a T-helper cell, then one viable method of detection is to employ a mixture of antibodies, as the detection markers, specific toward those antigens specific for T-helper cells, such the CD4 antigen (the "marker"). The primary antibody specific for, in this example the CD4 antigen, can be labeled using a fluorescent dye or a radioactive compound. Alternatively, a secondary antibody specific for the primary antibody can be labeled and used in the well known sandwich technique which amplifies the signal, as compared to just using a labeled primary antibody. Both of these labels can be detected using well known analytical instruments.

Plate reading devices are well known in the art. These commercial plate readers can analyze a conventional plate, such as a 96 well plate. These plate readers will analyze predetermined wells and generate raw data. This data can then be transformed and presented in a variety of ways.

Data can be obtained for one or more samples by manually removing the platforms that contain them from the block holding them, and presenting the platforms to the particular analytical device being used (e.g., fluorescence spectrometer). One embodiment uses a mechanical system (such as an automated robotic arm) to select, or "cherry-pick," particular platforms (e.g., those identified as satisfying certain criteria by the vision station) from the block(s) that contain them.

In one embodiment of the invention, cell markers are used to detect and/or characterize conditions that can be used to determine different cell states. In this embodiment, a platform (such as a 96 well plate) comprising cells and various cocktails is presented to a fluorescent spectrometer after a sufficient incubation time and is imaged. After each image capture, an analysis is performed to determine where the "areas of interest" in a platform are, where "areas of interest" can include cell populations, and in some instances, any remaining droplets of solution or solvent.

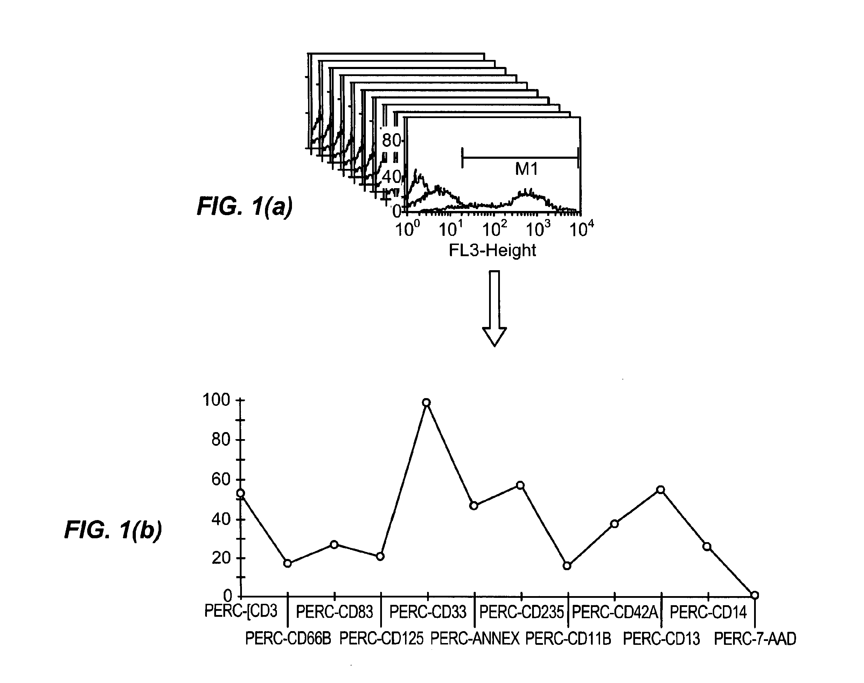

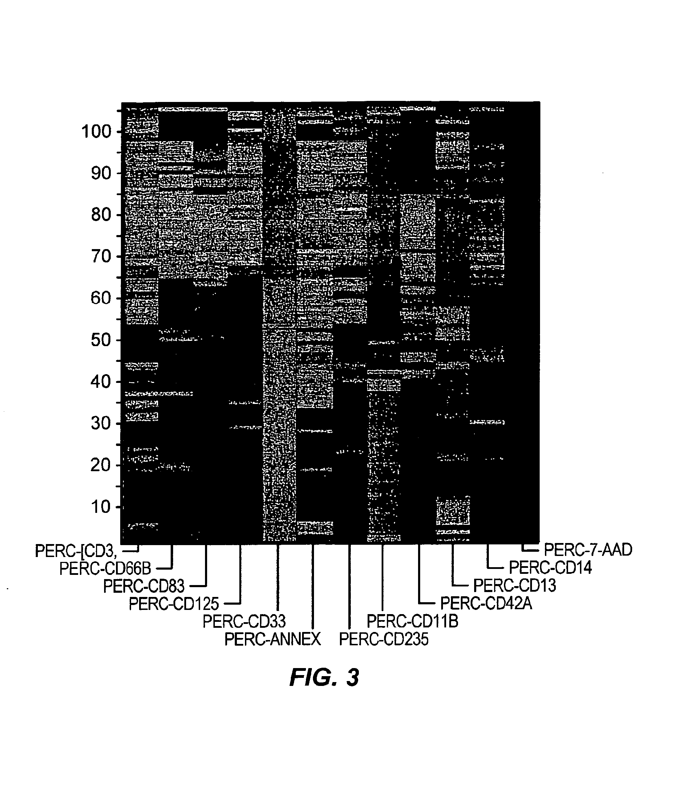

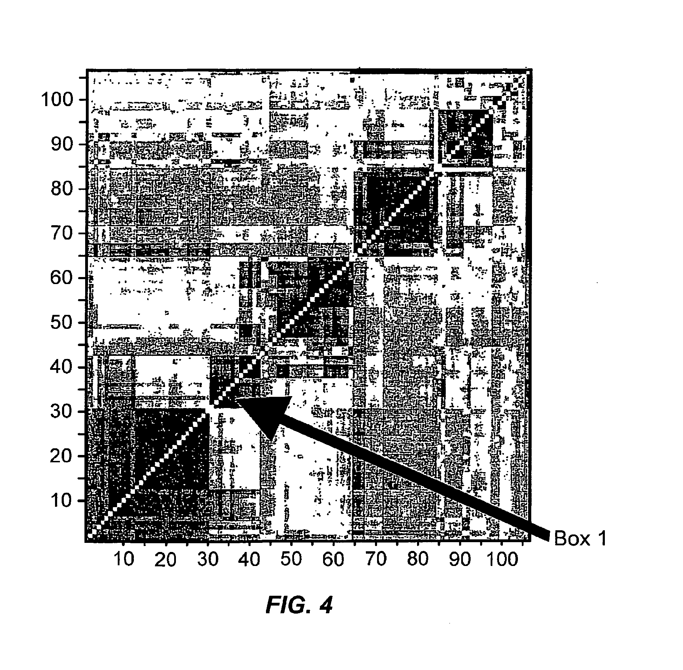

One type of marker analysis tool is spectroscopic analysis. Spectroscopic analysis of the platform will generate histograms. See FIG. 1. These histograms (FIG. 1a) reflect signal intensity for each marker per sample well, for example, four labeled detection markers can be used per well. The histograms are then transformed into a profile (FIG. 1b). A profile represents data obtained from one set of agents and one set of detection markers in which the cells were treated. The profile shows the results of multiple sample wells for a given experimental condition. The results are in terms of a marker response, *i.e*., whether, and to what extent, a given population of cells responded to a particular set of agents reflected in terms of marker intensity. Profiles have an "x" and "y" axis, wherein the "x" axis represents the markers being examined, and the "y" axis represents the percent positive of cells responding to a given detection marker. The profiles are then grouped to form a matrix (Figure 3). Each miniature box in the matrix represents a different experimental condition (e.g. a different set of agents). A matrix represents a grouping of conditions that favors a certain outcome reflected by response to the detection markers used. The matrices are then further analyzed and transformed into hierarchical clusters (Figure 4). The hierarchical clusters are clustered based on similarity to a specific profile. Thus, the boxes along the diagonal represent different cell states. In Figure 4, there are 5 different cell states. Thus, in box 1, there are multiple different combinations of agents which induced a cell state with a similar profile. Analysis can then be conducted on the data to determine which agents, combinations of agents, or lack of agents contributed towards a specific cell state. Only be creating a profile can a complex analysis of different cell states occur.

A profile can comprise as little as two markers or many more markers. A profile can comprise more than 3 markers, more than 4 markers, more than 5 markers, more than 8 markers, more than 10 markers, more than 15 markers, more than 20 markers, more than 25 markers, more than 30 markers, between 2 and 30 markers, between 2 and 20 markers, between 4 and 10 markers, between 3 and 8 markers, or between 5 and 50 markers. The number of markers needed depends upon the desired cell state. For example, to accurately characterize a specific hematopoietic stem cell which is actively dividing, a researcher may want to characterize this cell state with 8 different markers. Alternatively, other situations could arise where only two markers are desired. As an example, an experiment testing many different agents to look for induction of two specific proteins or genes could be done. In this situation, the complexity of testing multiple different agents is addressed by having a profile with two markers. Characterizing cell populations with additional markers allows characterization of specific cell states and not just different cell types. For example, typical characterization of hematopoietic stem cells may label cells as having the CD34 marker, but not having the CD33 marker. Characterizing the hematopoietic stem cells with 3, 5, 10, or 15 different markers could allow a greater understanding of the different cell states within the CD34+, CD33- population. Methods of this invention enable this characterization.

In particular embodiments of the invention which uses spectral analysis, spectroscopic data is processed using what is referred to herein as a "spectral binning system," which allows the rapid analysis and identification of samples in an array by creating, for example, a family or similarity map (or matrix) based on a particular profile. Some embodiments of the spectral binning system comprise a hardware-based instrumentation platform and a software-based suite of algorithms. The computer software is used to analyze, identify and categorize groups of samples having similar profiles, thus identifying a group from which the operator, or scientist, can then select a few samples for further analysis. This selection can be, performed independently by the scientist or using an automated means, such as software designed to automatically select samples of interest. Particular binning and analytical methods useful in the invention are disclosed in U.S. patent application Ser. No. 10/142,812, filed May 10, 2002.

The spectral binning system is generally used in this invention to detect similarities in the profiles of samples by observing their binning behavior. Thus, the number of cell populations demonstrating positive results to any given marker or combinations of markers can be estimated by binning spectra. The plurality of samples is examined with a device for generating a corresponding spectrum of acceptable quality, i.e*.,* sufficient S/N ratio. Advantageously, the profiles are compared pairwise in accordance with a metric to generate a similarity score. Other comparisons that use more than two spectra concurrently are also acceptable, although possibly complex.

One or more clustering techniques can be used to generate bins that are preferably well defmed, although this is not an absolute requirement since it is acceptable to generate a reduced list of candidate populations for a given set of conditions as an estimate of the heterogeneity of the conditions. Advantageously, the generation of bins facilitates the ready evaluation of cell populations among sample conditions.

The invention also encompasses the use of hierarchical clustering to represent the data in the form of a similarity matrix having similar profiles listed close together. Such a similarity matrix may be sorted to generate similarity regions along a diagonal. The hierarchical clustering algorithm uses the Euclidean distance between the spectra to obtain a (dis)similarity measure. Ordering the measures between all spectra from the experiment gives a Tartan plot, where each cluster is indicative of a possible cell state.

Advantageously, although the clusters are actually in higher dimensional space, they can be projected into 2 or 3 dimensional space and visualized. Preferably, the turn-around time for generating a profile and assigning the profile to a bin is less than about two minutes, one minute, ten seconds, or one second. Moreover, limited real time processing is often possible if an acquired profile is to be assigned to existing bins, or, in one embodiment of the invention, a library of binned profiles is updated with newly acquired profiles. In one embodiment, newly acquired profiles from a single sample may all be binned into a single bin based on a majority of them being more related to the single bin in accordance with a metric, such as those discussed below and elsewhere herein.

Once the profiles from all of the samples to be analyzed have been collected, they are processed by a series of algorithms. These algorithms facilitate the binning of sample profiles according to one or more spectral features. Examples of such features include, but are not limited to, percent positive for specific markers, the locations of peaks, peak shoulders, peak heights, and peak areas. In one embodiment, the spectral binning process bins profiles based on the percentage of positive cells in a well per condition examined, expressed as percent positive.

The process of finding peaks in a profile is an essential aspect of many spectral processing techniques, so there are many commercially available programs for performing this task. The many variations of peak finding algorithms can be found in the literature. An example of a simple algorithm is to find the zero-crossings of the first derivative of a smoothed or unsmoothed spectrum, and then to select the concave down zero-crossings that meets certain height and separation criteria.

In order to create these binary spectra, profiles are clustered with respect to percent positive per marker. The process used to perform this profiles clustering can be a modified form of a 1-dimensional iterative k-means clustering algorithm. The process begins with the spectra picked from a composite spectrum. A spectral bin covers a range of cells characterized by a particular profile that may be specified by the operator.

Using the similarity matrix or the binary profiles matrix, several different clustering methods can be employed to assign profiles into bins. Hierarchical clustering, k-means clustering, Gaussian mixture model clustering, and self-organizing map (SOM) based clustering are just some of the methods that can be used. These and other methods are well described in the literature. See Kohonen, T., "Self-organizing Maps", Springer Series in Information Sciences, Vol. 30, Springer, Berlin, Heidelberg, New York, 3rd Extended Edition (2001); Duda, R., Hart, P., and Stork, D., "Pattern Classification", John Wiley & Sons, 2.sup.nd Edition (November 2000); and Kaufman, L., Rowseeaww, "Finding Groups in Data", John Wiley & Sons, (1990), the entire teachings of which are incorporated herein by reference. In one embodiment, hierarchical clustering is used as a first-pass method of data analysis.

Using the information from the hierarchical clustering run, k-means clustering can then be performed with user-defined cluster numbers and initial centroid positions. In another embodiment, the number of clusters can be automatically selected in order to minimize some metric, such as the sum-of-squared error or the trace or determinant of the within cluster scatter matrix. See, Duda, R., Hart, P., and Stork, D., "Pattern Classification", John Wiley & Sons, 2nd Edition (November 2000), the entire teaching of which is incorporated herein by reference.

Hierarchical clustering produces a dendrogram-sorted list of profiles, so that similar profiles are very close to each other. This dendrogram-sorted list can be used to present the similarity matrix in a coded manner, wherein similarity indicia are used for each similarity region, including without limitation different symbols (such as cross-hatching), shades of color, or different colors. In a specific embodiment, the coded similarity matrix is presented in a color-coded manner, with regions of high similarity in hot colors and regions of low similarity in cool colors. Using such a visualization, many clusters become apparent as hot-colored square regions of similarity along the matrix diagonal. These square regions represent the high degree of similarity between all of the profiles in those regions. However, it should be noted that the failure of the coded similarity matrix to present a diagonal form is to be expected with some types of samples, although the matrix is still useful in representing more complex similarity relationships. Furthermore, in some cases there can be similarity regions along more than one possible diagonal that correspond to different rearrangements. Such rearrangements result in off-diagonal similarity square regions becoming part of the diagonal similarity square regions.

Along with the matrix representation of the cluster data, it is also useful to show where all of the profiles and the cluster boundaries lie in a dimensionally reduced space (usually 2-dimensions). There are several ways to perform this dimensionality reduction. In one embodiment, a linear projection is made of a binary profiles matrix onto its first two principal components. Alternatively, the chosen similarity matrix could be used in order to create a map of the data using multidimensional scaling.

In one embodiment, methods are directed toward the screening of multiple conditions for their ability to induce changes in cell state. In this embodiment, cells are incubated under suitable conditions and subjected to different experimental cocktails. After an appropriate amount of time, the cells are assayed to determine what, if any, marker characteristics they possess. In one aspect, early developmental stage cells, e.g., stem cells, are subjected to multiple conditions to determine what conditions facilitate the differentiation of these cells into more mature cells. In a particular aspect, the differentiated cells will elaborate specific antigens (or markers) on their cell surface which can be detected by a detection marker, such as an antibody specific for that antigen. In another aspect, cells are transfected or designed with a reporter gene. This reporter gene functions as the marker. Thus, agents which promote, inhibit, permit, or maintain the specific gene attached to the reporter gene can be characterized.

In another aspect, differentiated cells are subjected to various agents to determine which set of conditions results in the de-differentiation of a mature cell. Again, detection can be accomplished by detecting specific markers elaborated on an early stage cell, alternatively, the detection can be based on the loss of a particular marker(s) attendant to only mature cells.

One example of a method of the present invention involves high-throughput screening using multiple agents, multiple detection markers, creation of profiles, and data analysis. In one embodiment, the method involves the following informatics components: a DOE tool, a Tecan station controller, a flow cytometer, and a result viewer. In one aspect of the present embodiment, the following hardware components are employed: (1) a sterile Tecan (w/PC), and (2) a FACSCalibur Flow Cytometer.

The agents to be used in forming experimental conditions can be dissolved in appropriate solvents, such as DMSO (dimethyl sulfoxide) or ethanol. Appropriate concentrations are determined for differentiation factors and the factors are accordingly diluted in cell growth media to a concentration of Nx (where N is the order of the experiment- for a binary experiment N = 2, for a ternary N = 3, etc).

Once the agents and agent concentrations have been determined, a practitioner can design an experiment using a web-based DOE tool. This tool currently allows a practitioner to design a full factorial combinatorial experiment. The practitioner specifies the cell type that is being tested, the number of concentration per agent, the number of agents in a mixture, and the number of controls. Then the practitioner chooses appropriate agents and enters the concentrations that are to be tested. Finally, the practitioner submits the design and it is entered into the database.

The agents can be loaded onto, for example, a Tecan deck in 50 mL Falcon tubes. The Tecan station controller specifies the manner in which the Falcon tubes are to be loaded onto the deck. Covered, barcoded 96-well culture plates can also loaded onto the Tecan deck. The Tecan station controller generates a pipetting worklist that can be loaded into Gemini and run as part of a larger Tecan script. This Tecan script must remove the lids from the culture plates, perform the dispensing, and then puts the lids back on the plates.

In a specific example, the target volume for a full well is 200 µL. This process is repeated as many times as necessary to produce the appropriate number of plates for all the markers that are used in the experiment. Once the combinatorial dispense is complete, 5-10 µL of cells in cell growth media is added to all of the wells. A sterile Multidrop can be used for the dispensing. Then, the culture plates are incubated for two days. Sometime before the end of the second day of incubation, the initial dispense step is repeated exactly as described above. After 2 days of incubation, the original master culture plates are spun down. Media is gently removed and replaced with the fresh media from the second dispense. After 2 days additional days of incubation, the original master culture plates are labeled with detection markers. Agents can be added to the wells at various times. For example, agent can be added with the cells original plating, or the agent can be added just an hour before addition of the detection markers.

In fluorescence is used to detect a detection marker, wells of stained cells can be transferred to flow cytometry tubes and read on the flow cytometer. The wells are read in a column-first fashion (A1, B1, etc). Data files for a plate are stored in a directory with the plateID as a name. Well A1 is stored in a file named Barcode.001. Well A2 is stored in a file named Barcode.002, and so on. Once an entire plate has been read on the flow cytometer, the flow cytometer station controller can be used in order to load all the flow cytometer information into a database. A SpectraMax reader may be used in order to gather more information about cell cultures. ELISAs may be performed and the SpectraMax may collect either fluorometric or colorimetric endpoint data. A SpectraMax station controller will allow for SpectraMax data to be stored appropriately in the database.

The analysis application will incorporate all information for a given well or for a given agent into a single row. Thus, the analysis application will present both mixture-centric and well-centric views of data. Flow cytometry data for a row will be viewable through a 4D scatterplot. Flow cytometry data for 2 different rows will be able to be opened at once for comparison.

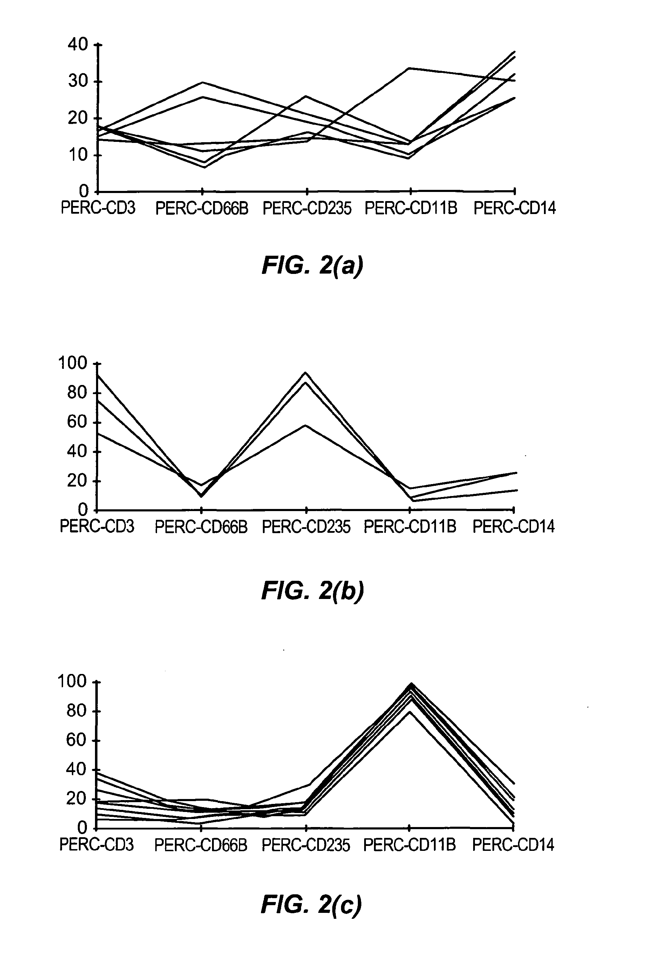

Figure 1(a) is an example of the type of data that is obtained. Single histogram plots are combined to create a profile (Figure 1(b)). This single profile represents one experimental condition (e.g. set of agents) and 12 different markers. Figure 2 (a-c) are illustrative of the data obtained in an experiment under multiple conditions. Profiles (a)- (c) represent data transformed from histograms that were collected from experiments conducted using different conditions on cells and detecting the same set of five markers on the surface of the cell. Each line in the three figures represents a different experimental condition. Figures 2(b) and 2(c) illustrate that even under different experimental conditions, cells can show similar profiles. The Figure 2(a) example illustrates that in other cases, cells will have different profiles which cannot be easily matched with other profiles. The three spectra represent three different clusters (or conditions). This type of analysis can identify certain experimental conditions which favor the elaboration of certain markers, sets of markers or profiles. Certain profiles indicate a specific cell state. Thus, this analysis can indicate a particular cell state or find conditions which promote, maintain, permit, or prohibit a certain cell state.

One embodiment comprises a method for identifying a cell state comprising the steps of: a) providing a cell population; b) introducing a set of agents to the cell population; c) detecting a set or markers; and d) creating a profile. The cells can be heterogeneous or homogeneous. The cells can be of any type sufficient to complete an assay. The number of agents can vary from two to 20 or more. In some aspects, the agents are tested in multiple different concentrations.

In another embodiment, a profile is created comprising the steps of choosing a set of markers, detecting the markers on a population of cells, and creating a graphical representation of the percent of cells expressing a particular marker. Alternatively, the graphical representation can show the total number of cells expressing said markers.

Another embodiment comprises a method of inducing a specific cell state comprising the steps of: a) identifying a desired cell population; b) creating a profile for said desired cell population, c) creating a cell population induced with a set of agents; d) identifying a profile for said cell population induced with said set of agents; and e) comparing said specific profile for said desired cell population to said profile for said cell population induced with said set of agents.

In another embodiment, a method of identifying conditions which induce a specific cell state comprises the steps of: a) identifying a desired cell population; b) creating a specific profile with a specific set of markers for said desired cell population; c) incubating a cell population with a set of agents; d) identifying a profile with the same set of markers for said cell population induced with said set of agents; and e) comparing said specific profile for said desired cell population to said profile for said cell population induced with said set of agents.

In a further embodiment, a profile comprises an x-axis representing at least two markers, and a y-axis representing percent positive cells responding to or expressing said markers. Alternatively, the x-axis of the profile could represent at least 3 markers, at least 5 markers, at least 8 markers, at least 10 markers, at least 15 markers, between 2 and 20 markers, between 4 and 10 markers, or between 3 and 8 markers. The x and y axis representations may also be reversed. In another embodiment, the percent positive cells are calculated by averaging the percent of cells expressing a particular marker from at least two test populations of cells. In another embodiment, said markers are identified by one or more detection molecules. The detection molecules may respond to spectroscopic analysis. Alternatively, the y-axis could represent the number of positive cells responding to the markers.

In another embodiment, a method of identifying similar cell states comprises the steops of creating a profile and grouping similar profiles. In some embodiments, the grouping occurs by hierarchical clustering.

Another embodiment entails the unexpected synergy of two agents. Phorbol-12-myristate-13-acetate (PMA) and dimethyl sulfoxide (DMSO) were shown to make a leukemia cell line more susceptible to apoptosis after treatment with an apoptosis promoting agent. In one embodiment, a composition contains PMA, DMSO and an apoptosis promoting agent. Specific examples of apoptosis promoting agents include anthracycline derivatives, idarubicin, and daunorubicin. Another embodiment entails a method of treating a patient with PMA, DMSO and an apoptosis promoting agent. Typical patients may include cancer patients, patients in need of leukemia cell apoptosis, breast cancer patients, leukemia patients, or patients typically treated with anthracycline derivative drugs. In another embodiment, a patient is first exposed to the PMA and DMSO and secondarily exposed to an apoptosis promoting agent. In another embodiment, a composition comprises a dosage form which first releases PMA and DMSO and secondarily releases an apoptosis promoting agent.

### EXAMPLES:

### Example 1: Binary & ternary experiments to examine differentiation of a cell

HL-60 cells were used to study what factors are involved in cellular differentiation. At day 0, the cells were plated in wells using a 96 well plate at a seeding density of approximately 60,000 cells, appropriate cell media was added. (See, Tables 1 & 2 below.) At day 2, the media was aspirated from the wells and fresh media was dispensed into the wells. Cells were induced with the factors in Table 1 and 2. At day 4, the cells were harvested and labeled with antibody (see Table 3) for cytometry. The labeling was accomplished by washing the cells with PBS (phosphate buffered saline). Then gamma globulin was used to block non-specific binding sites. The gamma globulin treatment lasted for approximately 20 minutes at room temperatures on a rocker shaker.

The antibody cocktail concentration for each antibody used was based on manufacturer's instruction adjusted for final cell number. The antibody cocktail was incubated for 30 minutes at room temperature on rocking shaker. Following the incubation, the cells were washed using PBS. The cells were resuspended in 1% ultrapure methanol free formaldehyde and refrigerated until analysis. The cells were analyzed using a BD FACSCalibur equipped with a high throughput sampler (HTS) which has a dual laser excitation line: argon (488 nm) and red diode (635 nm).

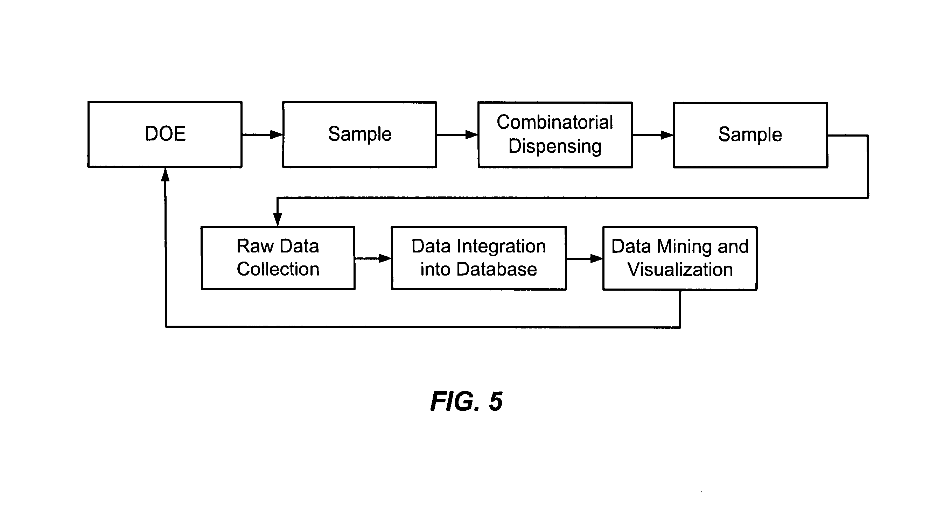

The positive control for these experiments was 100 nm vitamin D3 plus CD 14 antibody. The negative controls were (1) untreated cells plus antibody cocktails located on the plates; and (2) fresh untreated cells plus antibody cocktails located on separate plates. Figure 5 explains the analysis process.

**TABLE 1: binary experiment**

| Factor | Final conc. 1 | Final conc. 2 | Final conc. 3 |
|---|---|---|---|
| Vitamin D₃ | 100 nM | 1 nM | 10 pM |
| Dimethylsulfoxide | 0.26 M | 0.18 M | 0.13 M |
| All trans retinoic acid | 10 µM | 10 nM | 10 pM |
| Media pH 7.8 + sodium butyrate | 600 µM | 300 µM | 100 µM |
| 12-O-tetradecanoy-phorbol 13-acetate | 81 nM | 16 nM | 0.81 nM |

**TABLE 2: ternary experiment**

| Factor | Final conc. 1 | Final conc. 2 |
|---|---|---|
| Vitamin D₃ | 100 nM | 1 nM |
| Dimethylsulfoxide | 0.19 M | 0.15 M |
| All trans retinoic acid | 50 µM | 500 nM |
| Media pH 7.8 + sodium butyrate | 500 µM | 200 µM |
| 12-O-tetradecanoy- phorbol 13-acetate | 100 nM | 1 nM |

**TABLE 3: Antibodies and dyes**

| | | | | |
|---|---|---|---|---|
| CD 3 | CD 14 | CD 42 b | CD 66 a | CD 23 5 a |
| B220 | CD 33 | CD 56 | CD 72 | Annexin 5 |
| CD 11 b | CD 34 | CD 57 | CD 83 | 7-AAD |
| CD 11 c | CD 38 | CD 62 p | CD 86 | |
| CD 13 | CD 42 a | CD 66 | CD 125 w | |

### Example 2

HL-60 cells were exposed to five well studied chemical differentiation factors (dimethylsulfoxide (DMSO), Vitamin D₃, Phorbol-12-myristate-13-acetate (PMA), Sodium butyrate + pH 7.8, and all-Trans Retinoic Acid (ATRA)) known to promote differentiation along three distinct pathways (neutrophil, monocyte, eosinophil/basophil) within the myeloid lineage. Differentiation was induced by creating binary and ternary five factor combinations using the five factors at three concentrations for the binary experiment and two concentrations for the ternary experiment.

Following differentiation, morphological changes could be observed in wells containing combinations of differentiation factors as compared to control wells. For example, combinations containing PMA (16nM) + sodium butyrate (600 µM), pH 7.8, produced aggregates of cells while PMA (81 nM) + sodium butyrate (600 µM), pH 7.8 did not.

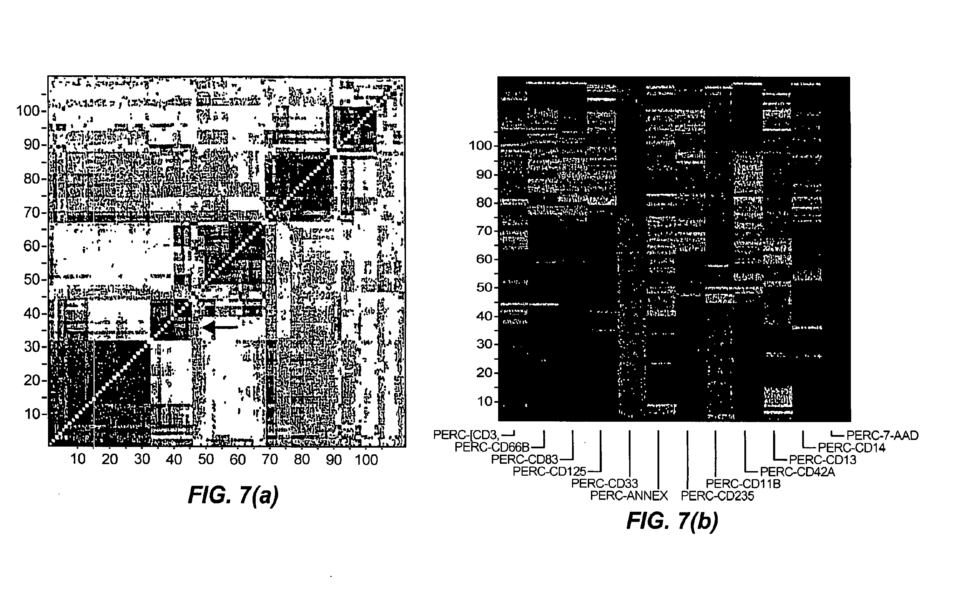

To observe experiment-wide profiles of marker expression, we used the percentage of positive cells for all markers and constructed a profile for each combinatorial treatment. Hierarchical clustering orders the data so that the most similar profile are next to each other in the plot. This results in several distinct partitions along the diagonal of the similarity matrix, and is shown in the Tartan plot in Fig. 7a. The spectra corresponding to the ordered treatments are shown in Fig 7b.

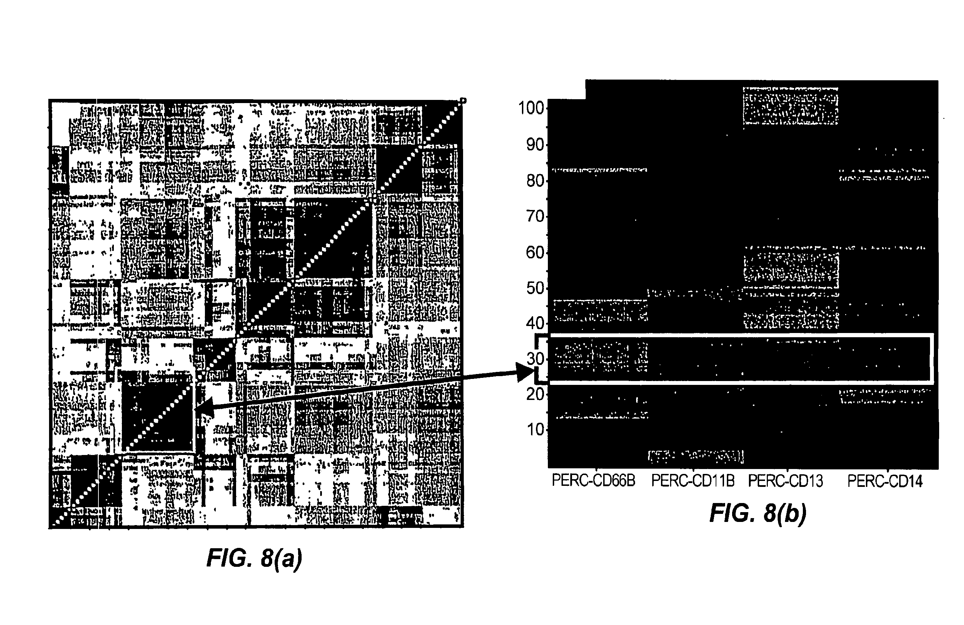

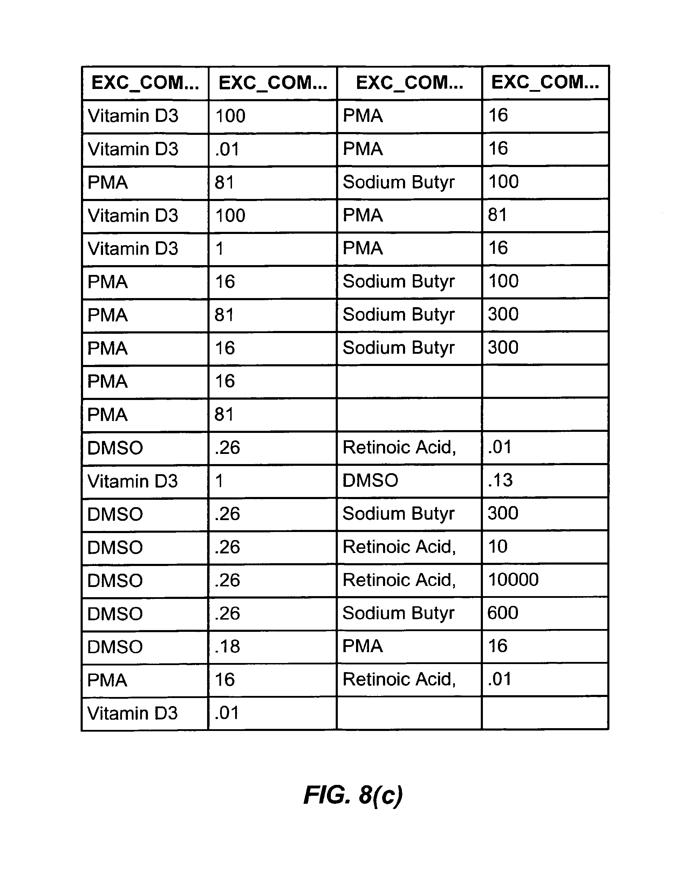

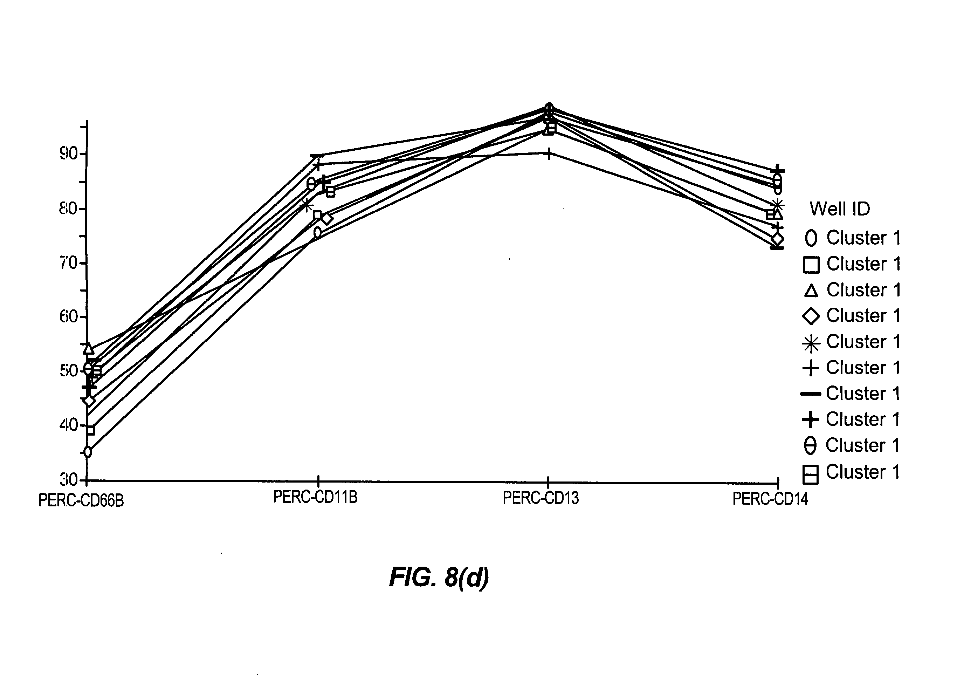

To refine cluster analysis with relevant information, one can choose specific cell surface markers. In order to test the validity of the experiment, we clustered based on cell surface markers known to be expressed in the myeloid lineage: CD66b, CD11b/Mac-1, CD13 +CD 14 (Figure 8 a,b). Cells which have differentiated toward the monocytic lineage have a CD 14^{hi}, CD11b/Mac-1^{hi}, CD 13⁺, CD66b⁻ expression profile. The spectral diagram for these surface markers shows that indeed there is a region which shows this expression pattern (Fig 4b, arrow, formulations)

By drawing a box around the corresponding formulations from the Tartan plot, we can click and view the formulation viewer to determine which factors contributed to the monocyte signature profile (Figure 8c). We determined that every treatment paradigm which contributed to the monocyte signature profile contains either Vitamin D₃ or PMA, two factors, consistent with literature findings, known to induce differentiation of HL-60 cells into monocytes. In addition, figure 8d shows the spectra for the phenotypic signature profile. Unexpectedly, it was found that the same or similar cell states can be induced under a variety of different conditions indicating the cells are entering a preferred cell state.

### Example 3

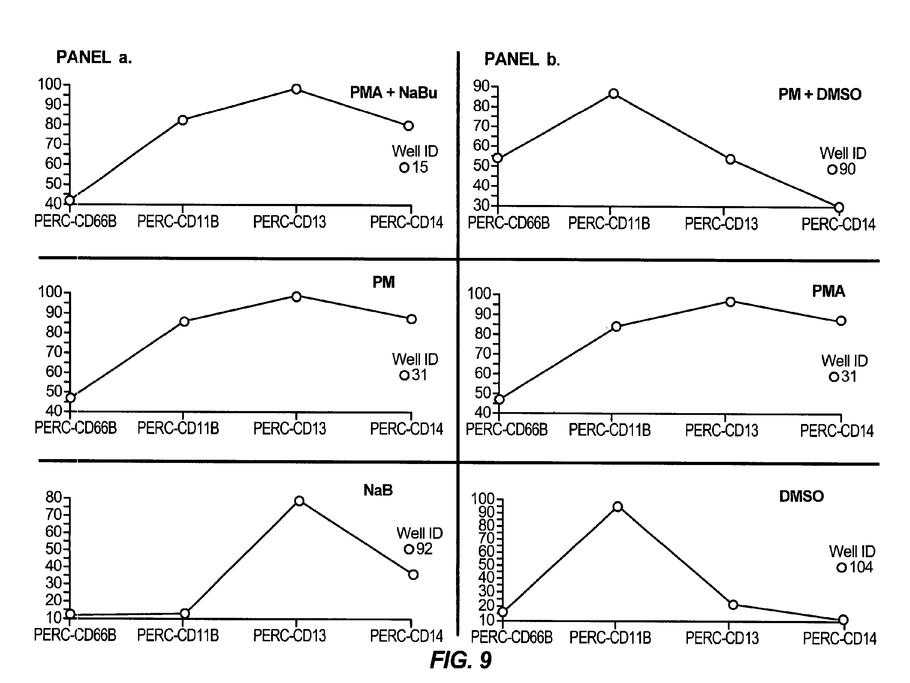

By querying the database, it was possible to find evidence of factor dominance. It was observed that when PMA + sodium butyrate (pH 7.8) were combined, a phenotype profile is produced which is most similar to that of PMA (Figure 9a). However, the presence of PMA as a dominating factor in one combination, does not always predict how that factor will behave in other treatment paradigms. For example, when DMSO is combined with PMA, the phenotype signature is most similar to that of DMSO, indicating that for this treatment paradigm, DMSO acts as the dominating factor (Figure 9b).

Evidence of non obvious interactions was found in which a profile for a combination treatment resulted in a unique profile compared to its individual components. The binary combination of DMSO^{med} + sodium butyrate^{med}, pH 7.8 produced cells expressing high levels of the cell surface marker CD 125w, whereas neither DMSO^{med} nor sodium butyrate^{med}, pH 7.8 alone produced cells positive for CD 125w. (The superscript notations "lo", "med", and "hi" are used to indicate relative concentrations of respective components.) The ternary combination consisting of DMSO^{lo}, sodium butyrate^{hi}, pH 7.8, and retinoic acid^{hi} produce a signature which has differential expression of CD 83 and CD 235 when compared to both the individual components and those in binary combinations. Furthermore, when DMSO, sodium butyrate, pH 7.8 and retinoic acid were combined in a different concentration scheme, a different signature profile was produced.

### Example 4:

In some cases, the combination of two differentiation factors produced unexpected surface marker expression. For example, some treatment paradigms produced cells which showed high expression of lymphocyte markers (CD3, B220), HSC markers (CD34) and erythrocytic markers (CD 235a). For most cases where high surface marker expression was observed in unexpected cell lineages, there was also high surface marker expression of myeloid lineage markers. The treatments producing this abnormal signature profile contained differing concentrations of PMA and DMSO.

HL-60 cells were induced to undergo differentiation for 5 days with either PMA, DMSO, or PMA + DMSO. The differentiated cells were then treated with Idarubicin, an anthracyclin derived antibiotic, commonly used to induce apoptosis in leukemic cells. Treatments containing PMA+ DMSO induced more cells to undergo apoptosis then either PMA or DMSO alone.

### NUMBERED EMBODIMENTS OF THE INVENTION

1. A method of identifying a cell state comprising: providing an array of receptacles each containing cells to be investigated; subjecting said cells in different receptacles to different agents; waiting a pre-determined period of time before analyzing said cells; analyzing said cells for expression of markers; creating a spectra representing marker expression of a cell population; and grouping said cell populations with similar spectra.
2. The method of embodiment 1, wherein said grouping is represented in a plot or graph.
3. The method of embodiment 1, wherein said cell state occurs in the presence of at least three different treatment conditions.
4. The method of embodiment 3, wherein said treatment conditions vary by the agent or agents added to the receptacles.
5. The method of embodiment 1, wherein said grouping occurs with the aid of a computer.
6. The method of embodiment 1, wherein said grouping occurs by: calculating the Euclidean distance between the spectra of different treatment conditions; and ordering the measures of distance of all spectra in a Tartan plot based on similarity.
7. The method of embodiment 1, further comprising the step of plotting cells with similar expression markers in profile comprising: a first axis representing at least two markers; and a second axis representing the number of positive cells expressing said markers.
8. A method of identifying a cell state, comprising the steps of: providing a cell population; introducing a set of agents to the cell population; detecting a set of markers; and creating a profile.
9. The method of embodiment 8, wherein said set of agents is at least 3.
10. The method of embodiment 8, wherein said marker is expression of a particular protein.
11. The method of embodiment 8, wherein a detection marker is used for detecting a set of markers.
12. The method of embodiment 11, wherein said detection marker is an antibody.
13. A method of inducing a specific cell state, comprising the steps of: identifying a desired cell population; creating a specific profile for said desired cell population; creating a cell population induced with a set of agents; identifying a profile for said cell population induced with said set of agents; and comparing said specific profile for said desired cell population to said profile for said cell population induced with said set of agents.

## Claims

1. A method for identifying a cell state comprising:
providing an array of receptacles each containing cells to be investigated,
subjecting said cells in different receptacles to at least 5 different agents,
waiting a pre-determined period of time before analyzing said cells,
analyzing said cells for expression ofmarkers,
creating a spectrum representing marker expression of a cell population; and
grouping said cell populations with similar spectra;
wherein said cell states are defined by at least 25 different treatment conditions having similar profiles.

2. The method of claim 1 wherein the cell states are identified by at least 50 or 100 different treatment conditions having similar profiles.

3. The method of claim 1 wherein the similar profiles are determined by calculating the Euclidean distance between the spectra of different treatment conditions; and ordering the measures of distance of all spectra in a Tartan plot based on similarity.

4. The method of claim 1 wherein the cell population is a heterogeneous cell population or a homogeneous cell population, and wherein the cell population is optionally derived from epithelial cells, endothelial cells, stem cells, mesenchymal cells, fibroblasts, neuronal cells, hematopoietic cells, or progenitor cells.

5. The method of claim 1 wherein the cell population wherein the agents are soluble factors; insoluble factors; cell matrix components; proteins; peptides; carbohydrates; small molecules; inorganic molecules; organic molecules, conditioned media, cell extracts, tissue extracts, pH modifiers, gasses, osmotic pressure modifiers; ionic strength modifiers; viruses; DNA; RNA; gene fragments; temperature modulators; mechanical stress modulators; or pressure modulators.

6. The method of claim 1 wherein at least some of the at least 5 agents are visible light, infrared light, ultraviolet light, ionizing radiation, hyperbaric pressure, increased or decreased temperature or pH, or acoustic vibrations.

7. The method of claim 1 wherein at least some of the at least 5 agents are administered to some receptacles in a different order than to other receptacles.

8. The method of claim 1 wherein the the marker is expression of a specific molecule; secretion of a specific agent; a specific phenotype; loss of a specific molecule; a change in membrane permeability; a change in electrical potential; cell death; cell migration; cell differentiation; gene expression changes; changes in protein levels; phosphorylation; methylation; or acetylation.

9. The method of claim 1 wherein the marker is an antibody, receptor; ligand, antisense molecule, small molecule, and reporter construct.

10. The method of claim 1 wherein the analyzing said cells for expression of markers comprises the use of a cocktail compositions comprising one or more detection molecules that is specific for a particular predetermined marker or set of markers.

11. The method of claim 1 wherein the analyzing said cells for expression of markers comprises use of a biological assay where said biological assay comprises one or more of transport of a compound across the cell membrane, electrical potential, action potential generation, cell proliferation, cell death, cell specification, cell differentiation, cell migration, gene expression or protein levels, enzymatic activity, phosphorylation, methylation, acetylation, changes in protein locus, ability to resist a pathogenic challenge, ability to respond to an agent, and ability to produce an immune response, and wherein the cocktail composition optionally comprises at least one antibody, nucleic acid binding molecule, receptor, ligand, antisense molecule, small molecule, or reporter construct specifically directed toward one of the expressed markers.

12. The method of claim 1 wherein the analyzing said cells for expression of markers comprises spectroscopic analysis to generate spectroscopic data, and wherein the spectroscopic analysis optionally further comprises providing a graphical profile correlating at least one set of agents with at least one set of detections markers in which the cells were treated, and wherein the data are optionally processed using a spectral binning system.

13. The method of claim 1 wherein the data are organized by a modified form of a 1 - dimensional iterative k-means clustering algorithm, hierarchical clustering, k-means clustering, Gaussian mixture model clustering, or self-organizing map (SOM) based clustering, and wherein optionally:
(a) hierarchical clustering is used to produce a dendogram-sorted list of profiles, then k-means clustering are performed with user-defined cluster numbers and initial centroid positions, using the information from the hierarchical clustering run;
(b) the number of clusters are selected in order to minimize the sum-of-squared error or the trace or determinant of the within cluster scatter matrix; or
(c) the grouping occurs with the aid of a computer or the grouping occurs by calculating the Euclidean distance between the spectra of different treatment conditions and ordering the measures of distance of all spectra in a Tartan plot based on similarity.

14. The method of claim 1 further comprising the steps of plotting cells with similar expression markers in profile comprising a first axis representing at least two markers, and a second axis representing the number of positive cells expressing said markers, wherein the agents optionally vary in one or more variables selected from the following:
a first agent variable relating to the number of agents added to said receptacle; a second agent variable relating to the timing of the addition of the agent into a receptacle; a third agent variable relating to the amount or concentration of agent added to a receptacle; a fourth variable relating to the identity or type of agent added into a receptacle; or a fifth variable relating to the period of time an agent is present in a receptacle.

15. A method of defining the conditions necessary to induce changes in a cell state comprising the method of claim 1 and further comprising identifying a cell population; creating a specific profile for said cell population; creating a cell population induced with a set of agents, identifying a profile for said cell population induced with said set of agents; and comparing said specific profile for said desired cell population for said cell population induced with said set of agents.
